# EUROPEAN PATENT APPLICATION

(11) **EP 4 290 221 A1**
(43) Date of publication of application: **13.12.2023**
(21) Application number: 22775013.0
(22) Date of filing: 04.03.2022
(51) Int. Cl.: G01N 21/64, C12M 1/34, C12Q 1/04, C12Q 1/70, G01N 21/03, G01N 21/78, G01N 33/569, G01N 37/00

(54) **VIRUS DETECTION METHOD USING WELL ARRAY, WELL ARRAY, AND DETECTION DEVICE**

(30) Priority: 26.03.2021 JP 2021053010
(71) Applicant: National Institute Of Advanced Industrial Science and Technology, Chiyoda-ku Tokyo 100-8921 (JP)
(72) Inventor: FUJIMAKI, Makoto, Tsukuba-shi, Ibaraki 305-8560 (JP); ASHIBA, Hiroki, Tsukuba-shi, Ibaraki 305-8560 (JP)
(74) Representative: Gille Hrabal Partnerschaftsgesellschaft mbB Patentanwälte
(86) International application number: PCT/JP2022/009368
(87) International publication number: WO 2022/202217

(57) **Abstract**

An object is to detect a virus with high speed and high sensitivity. Provided is a virus detection method including using a well array formed by defining a plurality of wells adjacent to each other by a side wall stood on a substrate and detecting a virus in a test liquid based on the detection of light emission from the wells, wherein the well array has a test region in which a well formation area, which is an area of the substrate in a region where the wells are formed, is 5 mm² or more, a total volume of the wells obtained by integrating individual volumes of the wells is 17 nL or more, a depth of the wells is 3.5 µm to 40 µm, an opening area of the wells is 18 µm² to 1,700 µm², a volume of the wells is 74 fL to 6,000 fL, and a thickness of the side wall between the wells adjacent to each other is 0.5 µm to 15 µm; and the detection of light emission from the wells is performed in a detection unit in which an observation visual field is fixed to have a size including the test region.

## Description

### Technical Field

The present invention relates to a virus detection method using a detection system in which the size of a detection element is set to perform high-speed and high-sensitivity virus detection; a well array, and a detection device.

### Background Art

As a protein detection method, a digital ELISA method (Enzyme-Linked Immuno Sorbent Assay) is known (refer to Non-Patent Documents 1 to 5, and Patent Documents 1 to 6), wherein, regarding a magnetic bead group which may include magnetic beads which have not trapped the protein and magnetic beads which have trapped the protein to which a labeled molecule having an enzyme is to be bound, the respective magnetic beads are moved and stored in microwells of a microwellarray having a large number of microwells arranged on a substrate, respectively, by using a magnet or gravity settling, and digitally counts the number of the microwells which have developed color. It is also reported that a virus-derived protein is detected using the digital ELISA method (refer to Non-Patent Document 6).

The microwell contains therein a fluorescent substrate and when the magnetic bead contained in the microwell has trapped the protein, that is, the protein is present in the microwell, color development (light emission) occurs due to an enzyme reaction between the enzyme and the fluorescent substrate and the presence or absence of the protein is observed as presence or absence of the color development of the microwell. At the same time, by digitally counting the number of the microwells causing color development (for example, by counting the microwell causing color development as "1" and the microwell not causing color development as "0"), the number of the virus in a sample can be detected and highly quantitative protein detection can be realized. When the present method is used for the detection of a virus protein as Non-Patent Document 6, highly quantitative virus detection can be realized.

However, in the digital ELISA method, the step itself of moving the magnetic beads one by one into the microwell by using the magnet or the gravity settling requires labor and time. In addition, when the labeled molecule has adsorbed to the magnetic bead without having the protein therebetween, color development occurs even in the microwell without the protein so that the unnecessary labeled molecule should be removed from the magnetic bead by preliminarily washing the magnetic bead to the extent that the trapped state of the protein is not damaged. This requires much labor and time for the detection operation. The digital ELISA method using the magnetic bead is therefore accompanied with the problem that it requires much labor and time for detection of the protein.

For these reasons, as a digital detection method not using the magnetic beads, there is proposed a method of trapping a virus directly in a microwell (hereinafter referred to as "direct virus trapping method") (refer to Patent Document 7 and Non-Patent Documents 7 and 8).

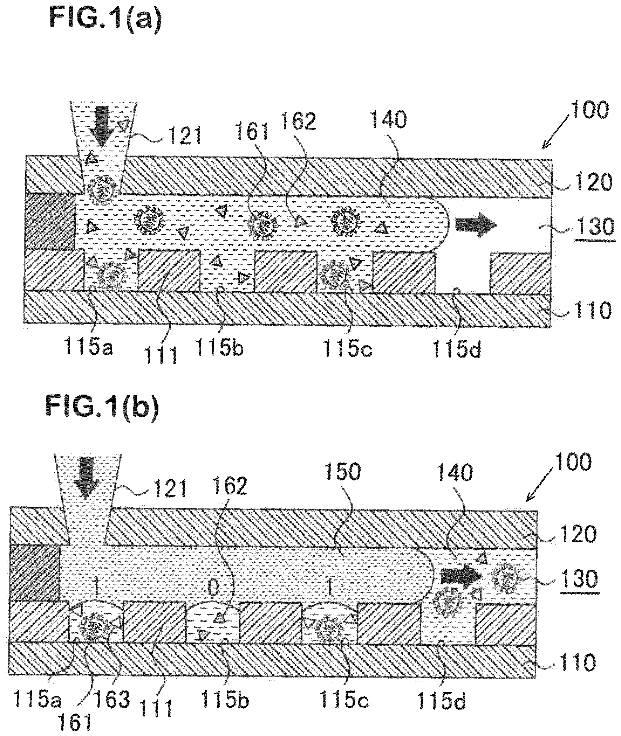

In this direct virus trapping method, detection is carried out, for example, by the procedure shown in FIGS. 1(a) and 1(b). FIG. 1(a) is a diagram (1) showing the detection procedure of the prior art and FIG. 1(b) is a diagram (2) showing the detection procedure of the prior art.

In this example of the direct virus trapping method, as shown in the drawings, a detection chip 100 having a space 130 between a lower-layer portion 110 and an upper-layer portion 120 is used.

In the lower-layer portion 110, side walls 111 each having a hydrophobized upper surface are vertically stood with a predetermined interval. Wells 115a to 115d are each formed as a minute space defined by side walls 111 adjacent to each other and an upper surface of the lower-layer portion 110, and the lower-layer portion 110 and the side walls 111 constitute a well array having a well arrangement of the wells 115a to 115d.

First, in the detection chip 100, a hydrophilic solvent 140 containing a virus 161 and a fluorescent color-developing substrate ((4-methylumbelliferyl)-α-D-N-acetylneuraminic acid) 162 reactive with an enzyme that the virus has is introduced into the space 130 from an introduction port 121 formed in the upper-layer portion 120 (refer to FIG. 1(a)).

Next, a hydrophobic solvent 150 (liquid hard to mix with the hydrophilic solvent 140) is introduced into the space 130 from the sample introduction port 121 formed in the upper-layer portion 120 and while pushing out the hydrophilic solvent 140 to the side of the well 115d, the hydrophilic solvent 140 is sealed in the wells 115a to 115c (refer to FIG. 1(b)). In short, the virus 161 is directly trapped in the well without using the magnetic bead.

In the well, with the passage of time, a reaction proceeds between the virus 161 and the fluorescent color-developing substrate 162 sealed in the minute space and a color developing reaction product 163 is formed (refer to FIG. 1(b)).

Therefore, color development due to the reaction product 163 occurs in the wells 115a and 115c having the virus 161 therein and the presence or absence of the virus 161 is observed as the presence or absence of the color development in the wells 115a to 115c. At the same time, by digitally counting the number of the wells in which color development has occurred, the number of the virus 161 in the sample is detected.

With respect to the size of the well in the detection chip 100, a distance between the side walls 111 adjacent to each other (a width-direction distance between the wells) is set very narrow. This assumes that if the distance is set wide, the color development of the reaction product 163 does not extend to the entirety of the well and thereby the color development of the wells 115a and 115c is blurred, so that it is impossible to distinguish these wells from the well 115b not having the virus 161 therein.

As a result, the volume of each of the wells 115a to 115c is set small. For that reason, when virus detection of a test liquid having a low virus concentration, that is, a test liquid containing a slight amount of the virus 161 in a large amount of a liquid is performed, a total volume of the wells 115a to 115c is less than the volume of the test liquid necessary for containing one virus 161 and a sufficient amount of the test liquid cannot be stored in the wells 115a to 115c, leading to a reduction in detection sensitivity.

On this point, the direct virus trapping method of the prior art eliminates the situation where the total volume of the wells is less than the volume of the test liquid by increasing the formation number of the wells. This means that detection sensitivity is secured by the formation number of the wells.

An increase in the formation number of the wells, however, increases an observation area and the resulting observation area exceeds an observation visual field in one observation.

Therefore, in the direct virus trapping method of the prior art, virus detection is performed, for example, by placing the detection chip 100 on a moving stage and changing the observation visual field a plurality of times to perform virus detection.

Virus detection performed by changing the observation visual field requires much labor and time for precise positioning during the change and integration of observation results obtained from a plurality of observation visual fields.

The direct virus trapping method of the prior art is therefore accompanied with the problem that the merit of detection without using the magnetic beads is damaged.

### Prior Art Documents

### Patent Documents

Patent Document 1: International Publication No. 2016/6208
Patent Document 2: Japanese Patent Laid-Open No. 2018-91737
Patent Document 3: International Publication No. 2012/121310
Patent Document 4: Japanese Patent No. 3886161
Patent Document 5: Japanese Patent No. 5551798
Patent Document 6: Japanese Patent No. 5363663
Patent Document 7: Japanese Patent Laid-Open No. 2018-38384

### Non-Patent Documents

Non-Patent Document 1: David M Rissin, et al., Nature Biotechnology Vol. 28, No. 6, pp.595(2010)
Non-Patent Document 2: Elena Perez-Ruiz, et al., Analytica Chimica Acta 1015, pp.74(2018)
Non-Patent Document 3: Soo Hyeon Kim, et al., Lab on a Chip 12, pp.4986(2012)
Non-Patent Document 4: David M Rissin, et al., Analytical Chemistry 83, pp.2279(2011)
Non-Patent Document 5: Stephanie M. Schubert, et al., Analytical Chemistry 88, pp.2952(2016)
Non-Patent Document 6: Karen Leirs, et al, Analytical Chemistry 88, pp. 8450(2016)
Non-Patent Document 7: Yoshihiro Minagawa, et al., Lab on a Chip 19, pp.2678(2019)
Non-Patent Document 8: Kazuhito V. Tabata, et al., Scientific reports 9:1067(2019)

### Disclosure of the Invention

### Problems to be solved by the Invention

An object of the present invention is to provide a virus detection method, a well array, and a detection device capable of detecting a virus with high speed and high sensitivity.

The present inventors have conducted intensive studies to solve the aforesaid problems and obtained the following findings.

First, the present inventors considered, instead of securing the detection sensitivity by the formation number of the well, securing the detection sensitivity by increasing the depth of the well and thereby eliminating the situation where the total volume of the well was less than a sufficient amount of the volume of the test liquid.

The target detection sensitivity is for virus detection in a test liquid having a virus concentration of 100 aM (one virus/17 nL) or less. Virus detection sensitivity at 100 aM is necessary for detecting the presence or absence of a virus from saliva as a screening test of a patient infected with the virus and is detection sensitivity demanded most at present for conducting a rapid virus test on many patients infected with the virus.

The baseline is to perform observation with a general-purpose observation device comprised of a 4x objective lens and an image pickup device of the full HD standard. From this baseline, an observation visual field on the side of a detection system is set at 5 mm². Using a 2x objective lens and a high-resolution image pickup device, the observation visual field may be set larger than 5 mm², but in order to establish a general-purpose detection method applicable even for the observation of many patients infected with a virus, the studies will be made herein based on the virus detection with the observation visual field of 5 mm².

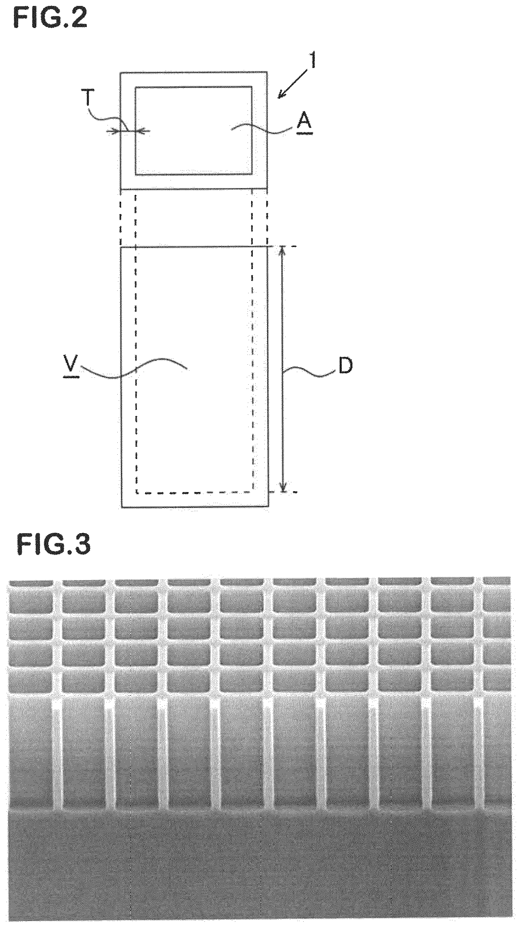

FIG. 2 shows an embodiment of a representative well to be studied. Further, FIG. 2 is an explanatory drawing for explaining the setting of the well, in which the upper side portion is the top view and the lower side portion is the front view.

In the well 1 shown in FIG. 2, setting targets are a depth D, a volume V, an opening space A, and a side wall thickness T.

### (Regarding depth D)

When the side wall thickness T is ignored, the lower limit of the depth D should be at least 3.4 µm (17nL/ 5 mm²) judging from the observation visual field (5 mm² or more) and a virus concentration of the test liquid (one virus/17 nL or less; a liquid amount necessary for detection is 17 nL or more).

In practice, due to the presence of the side wall thickness T, the depth D should be set more than 3.4 µm and at least 3.5 µm in order to eliminate the situation where the total volume obtained by integrating the volume V of the individual wells 1 is less than the volume of the test liquid.

On the other hand, when the depth D is too large, the image pickup device causes out of focus in the whole depth direction. In addition, the test liquid is hardly spread throughout the well 1. Further, the processing of the well 1 is made difficult.

The upper limit of the depth D should therefore be set at 40 µm at most.

### (Regarding volume V)

The present inventors manufactured a cubic-column well 1 having a depth D of 30 µm based on the aforesaid findings on the depth D and conducted a preliminary test for virus detection.

An electron microscopic image of the main portion of a well array including the well 1 used in the preliminary test is shown in FIG. 3.

In the preliminary test, a highly-concentrated virus-containing liquid diluted to a predetermined concentration was used as the test liquid. The virus used was an influenza A virus and (4-methylumbelliferyl)-α-D-N-acetylneuraminic acid was used as a fluorescent color-developing substrate that reacts with an enzyme in the virus to develop color.

In this preliminary test, virus detection was performed while changing the test conditions regarding the size of the well 1. As a result, it has been found that the virus detection results in failure when the virus concentration of the test liquid stored in one well 1 is less than one virus/6,000 fL.

The fluorescent color-developing substrate emits light slightly even in a liquid wherein the virus is not present. The virus concentration of the test liquid should therefore be high to a certain extent in order to distinguish whether the light emission in the well 1 is due to the light emission of the reaction product obtained by the reaction between the fluorescent color-1developing substrate and the virus or due to the light emission of the fluorescent color-developing substrate independent of the virus. The virus concentration which will be a detection limit when a material (the aforesaid fluorescent color-developing substrate or an aggregation-induced emission material, or the like) observed to emit light independently of the virus is used as a light emission source is estimated to be one virus/6,000 fL.

In order to distinguish the light emission in the well 1 having one virus trapped therein from the light emission independent of the virus, the amount of the test liquid to be stored in the well 1 should be 6,000 fL or less. Even if the test liquid having a virus concentration of 100 aM (one virus/17 nL) is used, when a volume V of the well 1 having one virus trapped therein exceeds 6,000 fL, a virus concentration of the test liquid in the well 1 reaches a detection limit, that is, less than one virus/6,000 fL, making it impossible to distinguish the above well 1 from another well 1 having no virus contained therein.

The upper limit of the volume V of the well 1 is therefore required to be set at 6,000 fL.

The lower limit of the volume V of the well 1 is, on the other hand, estimated as follows.

The pixel number in the full HD standard is 1,920 × 1,080. At least 3 × 3 pixels are necessary for imaging one well 1.

The total formation number of the well 1 for use in the general-purpose observation device is at most about 230,000, depending on the aforesaid pixel number (640 × 360).

Since the amount of a liquid necessary for realizing the virus detection at 100 aM is 17 nL, the volume V of one well 1 is required to be at least 74 fL based on the following relation: 17 nL/230,000 wells.

The lower limit of the volume V of the well 1 is therefore required to be set at 74 fL.

### (Regarding opening area A)

The upper limit of the opening area A of the well 1 is determined naturally by the depth D and the volume V of the well 1.

When the lower limit of the depth D is 3.5 µm, the opening area A is about 1,700 µm² to achieve 6,000 fL as the upper limit of the volume V.

The upper limit of the opening area A is required to be set at 1,700 µm². When the opening shape is square, the upper limit of the length of one side of the opening shape is about 41 µm.

On the other hand, the lower limit of the opening area A of the well 1 is estimated as follows.

The upper limit of the total formation number of the well 1 for use in the general-purpose observation device is 230,000 at most as described above. The baseline (lower limit) of the observation visual field is 5 mm².

When the side wall thickness T is ignored, the opening area A of at least 21.7 µm² (for example, an opening of 4.7 µm × 4.7 µm) is necessary per well 1 based on a 5 mm²/230,000 wells relation. Assuming that the opening area A when the side wall thickness T is ignored is less than 21.7 µm², the image of the well 1 becomes too small in an image pickup device of the full HD standard, making it impossible to distinguish individual wells.

In practice, however, the side wall thickness T exists. Assuming that the side wall thickness T is 0.5 µm (the reason will be described later), the opening area A of the well 1 is required to be at least 18 µm² (for example, a 4.2 µm × 4.2 µm opening).

The lower limit of the opening area A is required to be set at 18 µm².

### (Regarding side wall thickness T)

Regarding the upper limit of the side wall thickness T, when the side wall thickness is too large, a total volume obtained by integrating individual volumes V of the wells 1 is easy to be less than the volume of the test liquid so that it is required to be 15 µm at most.

The upper limit of the side wall thickness T is therefore required to be 15 µm.

Regarding the lower limit of the side wall thickness T, when the thickness is too small, processing becomes difficult and the resulting well tends to break easily.

The lower limit of the side wall thickness T is therefore required to be 0.5 µm.

As a result of the aforesaid studies, the present inventors have obtained findings on a virus detection method, a well array, and a detection device for carrying out high-speed virus detection at 100 aM with the general-purpose observation device.

### Means for solving the Problems

The present invention is based on the aforesaid findings and the following is means for solving the problems.
<1> A virus detection method including using a well array formed by defining a plurality of wells adjacent to each other by a side wall stood on a substrate and detecting a virus in a test liquid based on detection of light emission from the wells, wherein the well array has a test region in which a well formation area, which is an area of the substrate in a region where the wells are formed, is 5 mm² or more, a total volume of the wells obtained by integrating individual volumes of the wells is 17 nL or more, a depth of the wells is 3.5 µm to 40 µm, an opening area of the wells is 18 µm² to 1,700 µm², a volume of the wells is 74 fL to 6,000 fL, and a side wall thickness between the wells adjacent to each other is 0.5 µm to 15 µm; and the detection of light emission from the wells is performed in a detection unit in which an observation visual field is fixed to have a size including the test region.
<2> The virus detection method as described above in <1>, wherein the observation visual field of the detection unit is equal in size to the test region.
<3> The virus detection method as described above in <1> or <2>, wherein a luminescent material is adsorbed to a surface of the virus to cause the wells to emit light.
<4> The virus detection method as described above in <3>, wherein the luminescent material is an aggregation-induced emission material.
<5> The virus detection method as described above in <1> or <2>, wherein light emission from the wells is caused using a reagent that forms a fluorescent material by an enzyme reaction with a protein that the virus has.
<6> The virus detection method as described above in <1> or <2>, wherein light emission from the wells is caused using a reagent that forms a chemiluminescent material by an enzyme reaction with a protein that the virus has.
<7> A well array formed by defining a plurality of wells adjacent to each other by a side wall stood on a substrate and having a test region in which a well formation area, which is an area of the substrate in a region where the wells are formed, is 5 mm² or more, a total volume of the wells obtained by integrating individual volumes of the wells is 17 nL or more, a depth of the wells is 3.5 µm to 40 µm, an opening area of the wells is 18 µm² to 1,700 µm², a volume of the wells is 74 fL to 6,000 fL, and a thickness of the side wall between the wells adjacent to each other is 0.5 µm to 15 µm.
<8> A detection device including a detection chip in which the well array as described above in <7> is placed and a detection unit in which an observation visual field can be fixed with a size including the test region of the well array.
<9> The detection device as described above in <8>, wherein the observation visual field of the detection unit is set equal in size to the test region.

### Advantageous Effect of the Invention

The present invention makes it possible to overcome the aforesaid various problems of the prior art and provides a virus detection method, a well array, and a detection device capable of carrying out high-speed and high-sensitivity virus detection.

### Brief Description of the Drawings

FIG. 1(a) is a diagram (1) showing the detection procedure in the prior art.
FIG. 1(b) is a diagram (2) showing the detection procedure in the prior art.
FIG. 2 is an explanatory drawing for explaining setting of a well.
FIG. 3 shows an electron microscopic image of the main portion of a well array including wells 1 used in a preliminary test.
FIG. 4 shows an electron microscopic image in which a formation example of a well array is taken.
FIG. 5 shows an embodiment of a detection device.
FIG. 6 shows a fluorescent image in which a virus-containing specimen is taken in Example.
FIG. 7 shows a fluorescent image in which a virus-free specimen is taken in Example.

### Mode for carrying out the Invention

### (Well array)

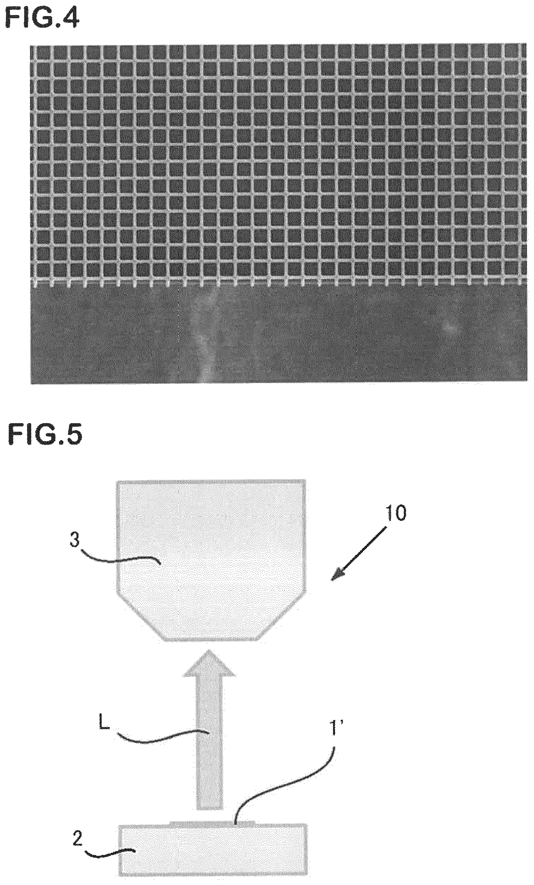

FIG. 4 shows an embodiment of the well array of the present invention. It is to be noted that FIG. 4 is an electron microscopic image in which a formation example of the well array is taken.

As shown in this electron microscopic image, the well array has a structure in which a plurality of wells are formed by defining the wells adjacent to each other with their side walls stood on a substrate.

A material for forming the well array is not particularly limited and can be selected as needed depending on the purpose. Examples thereof include glass materials, semiconductor materials, and resin materials.

A method of forming the well array is also not particularly limited and can be selected as needed depending on the purpose. Examples thereof include known methods such as a method of pattern drawing by lithography and then forming the well array by etching, a method of forming the well array by injection molding or imprint with a mold having a shape of the well.

For example, the processing limit of a method of forming the well array by lithography and reactive ion etching with silicon as a formation material is about 0.1 µm and thus the well array can be formed with high definition.

The upper surface of the side wall subjected to hydrophobic treatment is preferred from the standpoint of facilitating the test liquid to store in the well. The hydrophobic treatment method is not particularly limited and examples thereof include known methods such as application treatment of a hydrophobic polymer (for example, photoresist) and film formation treatment of a hydrophobic molecular layer (for example, dimethyldichlorosilane).

The well array shown in FIG. 4 is formed by lithography and reactive ion etching with a silicon substrate as a formation material.

The well array has a characteristic in the aforesaid conditions of the well formed by the above forming method. The conditions of the well will hereinafter be described while referring to FIG. 2 again.

### <Well formation area>

In the well array, a well formation area which is an area of the substrate in a region in which the wells 1 are formed in a test region is 5 mm² or more. It is to be noted that the well formation area indicates an area, on the substrate, in which a plurality of wells 1 are formed as a group of the wells 1 to be observed and is different from an area for forming one well 1.

The well formation area is set to be at least 5 mm² because it is based on the observation visual field in the case where the general-purpose observation device comprised of the 4x objective lens and the image pickup device of the full HD standard is used as a detection unit.

In other words, when a wider area is observed once, a larger number of wells can be observed once and a higher virus detection sensitivity can be achieved. When the observation visual field is less than 5 mm², image pickup should be performed a plurality of times to achieve high-sensitivity detection, which makes a detection step cumbersome. Therefore, the well array should be set based on that a light-emission state and a light-non-emission state in each well 1 formed in the well formation area of 5 mm² can be observed in the observation visual field of 5 mm².

The aforesaid detection unit may be comprised of a wide view field observation device. In this case, the observation visual field can be set larger while keeping necessary visibility.

For example, assuming that an image pickup device of the full HD standard (1,920 × 1,080 pixels) is used, the 2× objective lens is used, and the observation visual field is set at 20 mm², virus detection with higher sensitivity can be achieved in one observation. Further, if a 4K image pickup device (3,840 × 2,160 pixels) or 8K image pickup device (7,680 × 4,320 pixels) is used, image pickup of a wider area can be performed at an equal resolution and virus detection with high sensitivity can be achieved.

In this sense, the upper limit of the observation visual field may be about 320 mm² judging from the imaging limit of a known image pickup device. At the same time, from the standpoint of carrying out high-sensitivity detection by making use of the observation visual field without any waste, the upper limit of the well formation area is also about 320 mm², same as the area of the observation visual field. It is more preferred to set the upper limit of the well formation area at about 100 mm² (= 1cm × 1 cm), if ease of use, production cost and so on are taken into consideration.

### <Total volume of wells>

In the well array, the total volume of the wells 1 obtained by integrating the volumes of the individual wells 1 in the test region is set at 17 nL or more.

The reason why the total volume is at least 17 nL is to achieve the detection sensitivity at which virus detection from the test liquid having a virus concentration of 100 aM (one virus/17 nL) or less is realized with the general-purpose observation device. At such detection sensitivity, it is possible to detect the presence or absence of a virus from the saliva as a screening test of a patient infected with a virus.

Moreover, the aforesaid total volume can be set depending on the target virus concentration and the upper limit thereof is about 4,000 nL judging from the upper limit of the well formation area, the limit of the depth D of the well 1 described later, and so on.

### <Depth of well>

The depth D of the well 1 is set at 3.5 µm to 40 µm. When the depth D is less than 3.5 µm, detection sensitivity that realizes virus detection from the test liquid having a virus concentration of 100 aM (one virus/17 nL) or less cannot be achieved with the genera-purpose observation device. When the depth D is more than 40 µm, the image pickup device loses focus in the whole depth direction. In addition, the test liquid is hard to be spread throughout the well 1. Further, it makes the processing of the well 1 difficult.

### <Opening area of well>

The opening area A of the well 1 is set at 18 µm² to 1,700 µm². When the opening area A is less than 18 µm², the image of the well 1 becomes too small to discriminate individual wells when the general-purpose observation device is used. When the opening area A is more than 1,700 µm², on the other hand, the volume V of the well 1 becomes a volume at which detection reaches a limit when a material whose light emission is observed independently of a virus is used as a light emission source, making it impossible to distinguish the light emission from the well 1 due to the virus and light emission from the well 1 independent of the virus.

The opening area A of the well 1 is at least 18 µm² and this setting is based on the estimate when one well 1 is imaged with three pixels. When one well 1 is imaged with 4 pixels to have improved visibility, the lower limit of the opening area A of the well 1 is set at 25 µm².

In Example of the well 1 shown in FIG. 2, the opening has a square shape, but the opening shape is not particularly limited. It may be a regular polygon such as regular triangle and regular hexagon (honeycomb structure), a rectangle, a circle, or an ellipse. The well 1 is not particularly limited insofar as it has a columnar (square cup) shape.

### <Volume of well>

The volume V of the well 1 is set at 74 fL to 6,000 fL. When the volume V is less than 74 fL, detection sensitivity that realizes virus detection of the test liquid having a virus concentration of 100 aM or less cannot be achieved with the general-purpose observation device. When the volume V is more than 6,000 fL, the volume becomes a volume at which detection when a material whose light emission is observed independently of a virus is used as a light emission source reaches a detection limit, making it impossible to distinguish the light emission from the well 1 due to the virus and the light emission from the well 1 independent of the virus.

A clearer well image can be obtained by imaging of the well 1 at 4 × 4 pixels or more. In this case, the total formation number of wells 1 when the general-purpose observation device is used is about 130,000 at most, depending on the pixel number (480 × 270).

In this case, the volume V of one well 1 is required to be at least 130 fL based on the following relation: 17 nL/130,000 wells.

The lower limit of the volume V of the well 1 is therefore more preferably 130 fL.

### <Side wall thickness>

The side wall thickness T, that is, a thickness of the side wall between the wells 1 adjacent to each other is set at 0.5 µm to 15 µm. The side wall thickness T less than 0.5 µm makes the processing difficult and in addition, makes it breakable. When the side wall thickness T is more than 15 µm, on the other hand, the total volume is likely to be less than the volume of the test liquid. When the distance between the wells 1 adjacent to each other is not uniform as in the case where the well 1 has a round or oblong shape, the side wall thickness T is judged by the thickness of the thinnest portion between the wells 1 adjacent to each other.

When the well array has the constitution as described above, it is possible to achieve detection sensitivity that realizes, with the general-purpose observation device, virus detection of the test liquid having a virus concentration of 100 aM or less, by simultaneously satisfying various settings of the well 1 in the test region. In addition, it can be used for the virus detection by the aforesaid direct virus trapping method that does not use the magnetic beads and a virus can be detected in a short time. In addition, even one-minute virus detection can be realized under suitable conditions.

### <Test region>

The well array may have, in addition to the well containing the test liquid for detecting light emission, an arbitrary well which is positioned outside the observation visual field in the detection unit and is not subject to detection of light emission. For example, the area of the observation visual field in the detection unit in which the observation visual field is fixed is preferably equivalent in size to the area of the formation region of the well that contains the test liquid for detection of light emission. From the standpoint of carrying out observation operation with room to spare during detailed alignment between the observation visual field and the formation region of the well to be observed, a region which will be a "backup margin" may be provided outside the observation visual field in the well array and the arbitrary well may be formed in this region.

Described specifically, without the arbitrary well, it takes time to align the observation visual field and the well formation region because the size of the well is minute. When the arbitrary well is present, on the other hand, alignment can be performed conveniently by making use of the visibility of the outline which can be recognized in the outermost arbitrary well. In other words, when the arbitrary well is present, alignment of the observation visual field and the well formation region can be achieved only by finding the outline and setting the observation visual field inside the outline. The arbitrary well may be formed to have a size similar to that of the well that contains the test liquid for detection of light emission or it may be formed, as an alignment adjusting well, to have a size different from that of the well that contains the test liquid for detection of light emission.

The well formation region to be observed is called "test region" herein and is distinguished from the region of "discarding margin" which is not a region to be observed. It is to be noted that in this sense, the terms "well formation area", "total volume of the well", "depth of the well", "opening area of the well", "volume of the well", and "thickness of the side wall" mean "well formation area", "total volume of the well", "depth of the well", "opening area of the well", "volume of the well", and "thickness of the side wall" in the "test region", respectively.

### (Detection device)

The detection device of the present invention has a detection chip in which the well array of the present invention is placed and a detection unit. An embodiment example of the detection device is shown in FIG. 5.

As shown in FIG. 5, a detection device 10 has a detection chip 2 in which a well array 1' having a plurality of the wells 1 formed therein is arranged and a detection unit 3.

The detection chip 2 is not particularly limited insofar as it has the well 1 and the well array 1' having the aforesaid characteristics. It can be formed based on a known constitution. For example, it can be formed based on the constitution of a detection chip 100 (refer to FIGS. 1(a) and (b)) having a space 130 between a lower-layer portion 110 and an upper-layer portion 120. It may also have, for example, a constitution obtained by sealing the upper portion of the well array 1' with a transparent resin or silicone rubber or a constitution obtained by adding dropwise a hydrophobic solvent to the upper portion of the well array 1' to seal the upper portion of the well 1 and placing a cover glass thereon.

In the detection unit 3, the observation visual field large enough to include the test region in the well array 1' is fixable.

This detection unit 3 is comprised based on the constitution of a detection unit used in a known microscope. For example, it is comprised of the general-purpose observation device having the 4x objective lens and the image pickup device of the full HD standard. Instead of the general-purpose observation device, the detection unit may be comprised of the wide view field observation device having the 2x objective lens and the 4K image pickup device, the 8K image pickup device, or the like.

The observation visual field of the detection unit 3 may be large enough to include the test region, but it is preferably equal in size to the test region.

Thus, when the observation visual field and the test region are set to have an equal size, the observation visual field can be used without any waste to achieve high-sensitivity detection.

When fluorescence is detected as light emission from the well 1, the detection device has a light irradiation unit for irradiating light to excite fluorescence. This light irradiation unit is constituted based on the constitution of a known light irradiation unit for fluorescence excitation and has, for example, a light source for fluorescence excitation selected according to the excitation wavelength of a material which causes fluorescence emission from the well 1. The light source may be a known one such as laser, LED, or lamp and if necessary, it may be a combination with a lens or optical filter.

By using the detection device having the constitution as described above and satisfying various settings of the well array simultaneously, it is possible to achieve, with the general-purpose observation device, detection sensitivity that realizes virus detection of the test liquid having a virus concentration of 100 aM (one virus/17 nL) or less. In addition, it can be used for virus detection by the direct virus trapping method not using the magnetic beads and virus detection can be achieved in a short time. Further, one-minute virus detection can also be realized under suitable conditions.

### (Virus detection method)

The virus detection method of the present invention is a method of using the well array of the present invention and detecting a virus in the test liquid based on the detection of light emission from the well. The detection of light emission from the well is performed at a detection unit in which the observation visual field is fixed to have a size including the test region.

To the detection unit, items similar to those of the detection unit 3 (refer to FIG. 5) described above for the detection device of the present invention can be applied.

The observation visual field of the detection unit may be large enough to include the test region and it is preferably as large as the test region.

When the observation visual field and the test region are set to be equal in size, the observation visual field can be used without any waste to achieve high-sensitivity detection.

A virus to be detected is not particularly limited and viruses detectable by a known virus detection method such as ELISA and immunoassay may be used. Examples include an influenza virus, a norovirus, and a coronavirus.

The method of detecting light emission from a well is not particularly limited and examples include a method of making use of an adsorption reaction, enzyme reaction or chemical reaction and thereby detecting light due to the presence of a virus. Specific examples include a method of adsorbing a luminescent material to the surface of a virus to cause light emission from the well, a method of using a reagent that produces a fluorescent material by an enzyme reaction with a protein in a virus to cause light emission from the well, and a method of using a reagent that produces a chemiluminescent material by an enzyme reaction with a protein in a virus to cause light emission from the well.

The luminescent material is not particularly limited and can be selected as needed depending on the kind of a virus. For example, an aggregation-induced emission (AIE) material is preferred. Examples of the aggregation-induced emission material include compounds producing an AIE effect as described in Japanese Patent Laid-Open No. 2010-112777.

The reagent that produces a fluorescent material by an enzyme reaction with a protein in a virus is not particularly limited and can be selected as needed depending on the kind of the virus. Examples include 4-methylumbelliferone-containing derivatives such as (4-methylumbelliferyl)-α-D-N-acetylneuraminic acid that enzymatically reacts with neuraminidase in an influenza virus and thereby produces 4-methylumbelliferone, fluorescein-containing derivatives, resorufin-containing derivatives, and rhodamine-containing derivatives.

The reagent that produces a chemiluminescent material by an enzyme reaction with a protein in a virus is not particularly limited and can be selected as needed depending on the kind of the virus. Examples include known artificial luciferin.

The detection of light emission from the well is performed by storing, in the well, the test liquid containing both a virus and a material (reagent) causing light emission from the well and then detecting the luminescence from the well at the detection unit.

A method of storing the test liquid in the well is not particularly limited. When the detection chip having a constitution based on the detection chip 100 (refer to FIGS. 1(a) and (b)) having a space 130 between a lower-layer portion 110 and an upper-layer portion 120 is used, examples include a method of preparing the test liquid as a hydrophilic solvent, introducing it in the space 130, introducing a hydrophobic solvent into the space 130, sealing the resulting test liquid in the well, and thus storing the test liquid in the well.

Examples further include a method of preparing the test liquid as a hydrophilic solvent, dropping the resulting liquid on the well array, pressing a transparent resin or silicon rubber onto the well array for sealing, and thus storing the test liquid in the well; a method of preparing the test liquid as a hydrophilic solvent, dropping the resulting liquid onto the well array, substituting the hydrophilic solvent on the surface with a hydrophobic solvent, placing a cover glass thereon, and thus storing the test liquid in the well.

According to the virus detection method performed as described above, by simultaneously satisfying various settings of the well array, detection sensitivity that realizes virus detection from the test liquid having a virus concentration of 100 aM (one virus/17 nL) or less can be achieved with the general-purpose observation device. Also, it can be used for the virus detection by the aforesaid direct virus trapping method that does not use the magnetic beads and a virus can be detected in a short time. In addition, even one-minute virus detection can be realized under suitable conditions.

### Examples

The following detection test was performed with an influenza virus as a detection target to confirm the validity of the virus detection method of the present invention.

In the detection test, there was used a well array obtained by forming 251 × 201 wells, 50,451 wells in total, on a silicon substrate, each well having a 7 µm-side square as an opening shape, a depth D of 10 µm, an opening area of 49 µm², and a volume of 490 fL, while setting a side wall thickness T at 3 µm. In the resulting well array, a well formation area was 5 mm² and a total volume of the wells was 25 nL. It was manufactured by a known shape processing method using photolithography and dry etching.

As the reagent for detecting the influenza virus, there was used a detection reagent (that synthesized by referring to H Shi, et al., Journal of the American Chemical Society 134, pp. 9569 (2012)) obtained by binding a peptide molecule that bound to the influenza virus to tetraphenylsilole, an aggregation-induced emission material.

As a camera for observing the well array, there was used a 2,048 × 2,048 pixel (4,194,304 pixels) CMOS camera (ORCA-Flash4.0 V3, manufactured by Hamamatsu Photonics K.K.). The observation visual field when observed with a 4x objective lens is 3.25 mm × 3.25 mm so that the whole well array can be observed.

The following is the details of the detection method. First, 30 µL of a sample liquid containing the influenza virus and 30 µL of an aggregation-induced emission material solution having a concentration of 0.4 µM were mixed and a 50 µL portion of the resulting mixture was introduced into the well array.

The well array was then sealed with a fluorine oil and covered with a cover glass, followed by observation with a fluorescence microscope (BXFM, manufactured by Olympus Corporation) through the 4x objective lens.

The influenza virus bound to the aggregation-induced emission material to cause fluorescence enhancement of the aggregation-induced emission material. In practice, the well having the influenza virus sealed therein was observed to be brighter than the well not containing the influenza virus.

To determine a luminescent well containing the influenza virus, a value obtained by standardizing the luminance (standardized luminance value) of the individual wells with an average luminance in a 200 × 200 pixel range of a portion outside the well array and not having the well array formed therein was used for the analysis.

A threshold value for determining the luminescent well was set at µ+3.3σ based on an average µ of the standardized luminance as measured with the sample not containing the influenza virus and a standard deviation σ. The number of the wells exceeding the threshold value was counted as the luminescent wells and it was used as a measurement value corresponding to the concentration of the influenza virus.

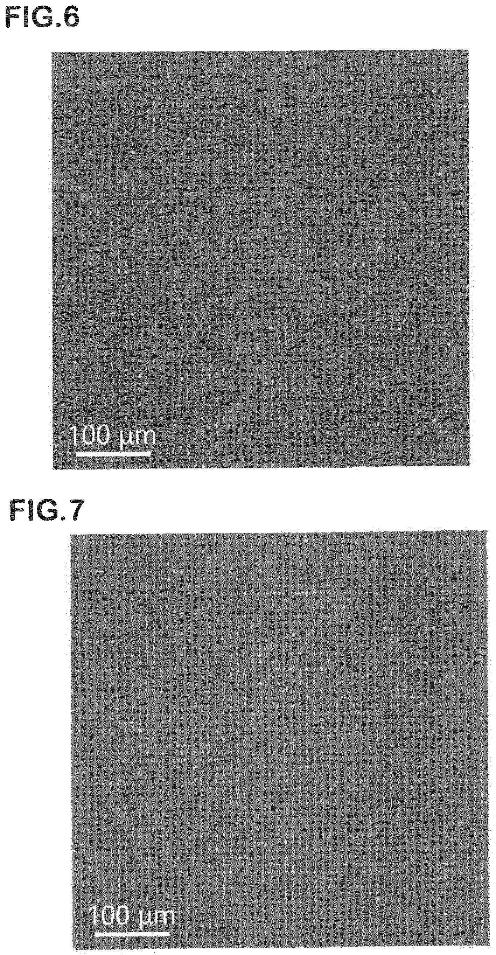

As a result of the above test, an example of a fluorescent image taken using a sample containing an influenza virus at a final concentration of 1 × 10⁵ copy/mL is shown in FIG. 6 and an example of a fluorescent image taken using the blank sample is shown in FIG. 7.

From comparison between FIG. 6 and FIG. 7, it can be confirmed that in the case of a sample containing the influenza virus, a well brighter (white) than the well therearound is observed, while in the case of a sample not containing the influenza virus, almost all the wells have an equivalent brightness.

In the whole well array, the number of the luminescent wells containing the influenza-virus-containing sample is 61 and the number of the luminescent wells containing the influenza-virus-free sample is 8, revealing that they can be discriminated significantly and detection of the influenza virus can be achieved. When the sample shows that the number of the luminescent wells is at least 20 or more, significant discrimination from the influenza-virus-free sample can be achieved. Further, the number of the luminescent wells is presumed to be proportional to the virus concentration so that a sample containing the influenza virus having a final concentration of up to about 3 × 10⁴ copy/mL can be subjected to virus detection.

The aforesaid final concentration (3 × 10⁴ copy/mL) is a concentration corresponding to 50 aM. It has been confirmed that the binding reaction of the influenza virus to the aggregation-induced emission material is completed within 10 seconds so that even if the time for sample introduction into the well array or fluorescence observation is included, the whole measurement step may be completed within one minute. In short, the present invention achieves high-speed and high-sensitivity virus detection.

### Description of Reference Numerals

- 1:: well
- 1':: well array
- 2:: detection chip
- 3:: detection unit
- 10:: detection device
- L:: light emission
- 100:: detection chip
- 110:: lower-layer portion
- 111:: side wall
- 115a to 115b:: well
- 120:: upper-layer portion
- 121:: introduction port
- 130:: space
- 140:: hydrophilic solvent
- 150:: hydrophobic solvent
- 161:: virus
- 162:: fluorescent color-developing substrate
- 163:: reaction product

## Claims

1. A virus detection method, comprising:
using a well array formed by defining a plurality of wells adjacent to each other by a side wall stood on a substrate; and
detecting a virus in a test liquid based on detection of light emission from the wells,
wherein the well array has a test region in which a well formation area, which is an area of the substrate in a region where the wells are formed, is 5 mm² or more, a total volume of the wells obtained by integrating individual volumes of the wells is 17 nL or more, a depth of the wells is 3.5 µm to 40 µm, an opening area of the wells is 18 µm² to 1,700 µm², a volume of the wells is 74 fL to 6,000 fL, and a thickness of the side wall between the wells adjacent to each other is 0.5 µm to 15 µm; and
wherein the detection of light emission from the wells is performed in a detection unit in which an observation visual field is fixed to have a size including the test region.

2. The virus detection method according to Claim 1, wherein the observation visual field of the detection unit is equal in size to the test region.

3. The virus detection method according to Claim 1 or 2, wherein a luminescent material is adsorbed to a surface of the virus to cause light emission from the wells.

4. The virus detection method according to Claim 3, wherein the luminescent material is an aggregation-induced emission material.

5. The virus detection method according to Claim 1 or 2, wherein light emission from the wells is caused using a reagent that forms a fluorescent material by an enzyme reaction with a protein that the virus has.

6. The virus detection method according to Claim 1 or 2, wherein light emission from the wells is caused using a reagent that forms a chemiluminescent material by an enzyme reaction with a protein that the virus has.

7. A well array formed by defining a plurality of wells adjacent to each other by a side wall stood on a substrate, comprising:
a test region in which a well formation area, which is an area of the substrate in a region where the wells are formed, is 5 mm² or more, a total volume of the wells obtained by integrating individual volumes of the wells is 17 nL or more, a depth of the wells is 3.5 µm to 40 µm, an opening area of the wells is 18 µm² to 1,700 µm², a volume of the wells is 74 fL to 6,000 fL, and a thickness of the side wall between the wells adjacent to each other is 0.5 µm to 15 µm.

8. A detection device, comprising:
a detection chip in which the well array as claimed in Claim 7 is placed; and
a detection unit in which an observation visual field can be fixed with a size including the test region of the well array.

9. The detection device according to Claim 8, wherein the observation visual field of the detection unit is set equal in size to the test region.
